(19) Europäisches Patentamt
European Patent Office
Office européen des brevets

(11) Veröffentlichungsnummer: **0 344 602 A1**

(12) **EUROPÄISCHE PATENTANMELDUNG**

(21) Anmeldenummer: **89109371.8**

(22) Anmeldetag: **24.05.89**

(51) Int. Cl.⁴: **C07D 309/30 , C07C 69/732 , C07C 59/48 , A61K 31/365**

(30) Priorität: **01.06.88 DE 3818570**

(43) Veröffentlichungstag der Anmeldung: **06.12.89 Patentblatt 89/49**

(84) Benannte Vertragsstaaten: **AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Dreckmann, Bruno, Dr.**
**Dürerstrasse 18**
**D-6800 Mannheim 25(DE)**
Erfinder: **Heck, Reinhard, Dr.rer.nat.**
**Wiesenstrasse 29**
**D-6836 Oftersheim(DE)**
Erfinder: **Pill, Johannes, Dr.rer.nat.Dr.med.**
**In der Keitgasse 6**
**D-6909 Leimen 3(DE)**

(54) **6-substituierte 4-Hydroxytetrahydro-Pyran-2-on-Derivate, Verfahren zu ihrer Herstellung, pharmazeutische Präparate auf der Basis dieser Verbindungen.**

(57) Verbindungen der Formel Ia und Ib

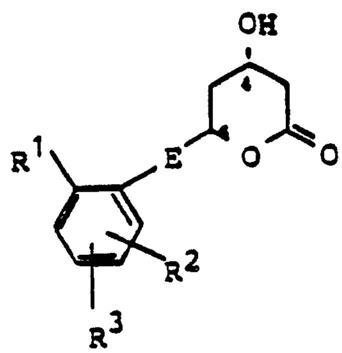

I a

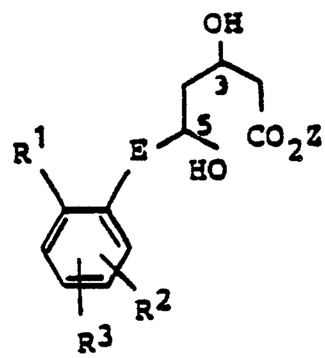

I b

in denen
E eine Alkylen- oder Alkenylen-Rest mit 2 Kohlenstoffatomen,
$R^1$ einen cycloaliphatischen Kohlenwasserstoffrest mit 5 bis 12 Kohlenstoffatomen, der ggf. mit bis zu 4 niederen Alkylgruppen sowie Halogen substituiert ist und ebenfalls ein- oder mehrfach ungesättigt sein kann,
$R^2$, $R^3$ die gleich oder verschieden sein können:
- einen weiteren cycloaliphatischen Kohlenwasserstoffrest mit 3 bis 7 C-Atomen,
- Wasserstoff,
- Halogen
- Trifluormethyl,

EP 0 344 602 A1

- Alkyl- oder Alkoxy-Rest mit 1 bis 7 C-Atomen, der ggf. noch mit Methylgruppen substituiert sein kann.
Z Wasserstoff, ein Alkalimetallion oder ein Ammoniumion bedeuten,
Ester und Amide der freien Carbonsäure, sowie deren stereoisomere Formen,
Verfahren zu ihrer Herstellung und Arzneimittel, die diese Verbindungen enthalten, zur Behandlung von Stoffwechselerkrankungen.

# NEUE 6-SUBSTITUIERTE-4-HYDROXY-TETRAHYDRO-PYRAN-2-ON-DERIVATE, VERFAHREN ZU IHRER HERSTELLUNG, PHARMAZEUTISCHE PRÄPARATE AUF BASIS DIESER VERBINDUNGEN

Der Hauptrisikofaktor für atherosklerotische Veränderungen der Koronararterien mit Herzinfarkt als Folge ist eine erhöhte Konzentration cholesterolhaltiger Lipoproteine geringer Dichte (LDL, low density Lipoproteins) im Plasma. Die Senkung des Cholesterinspiegels dient daher sowohl der Prophylaxe als auch der Therapie dieser multifaktoriellen Erkrankung (Lipid Research Clinics Programm, LRC-CPPT, J. Am. Med. Assoc. 251, 351 - 374; 1984).

Da ein großer Teil des Cholesterins auf die de novo-Synthese zurückzuführen ist, stellt die Kontrolle der endogenen Cholesterin-Bildung eine effektive Therapiemöglichkeit dar.

Das geschwindigkeitsbestimmende Schlüsselenzym für die Biosynthese ist die 3-Hydroxy-3-Methyl-Glutaryl-CoA-Reduktase. Dieses Enzym ist verantwortlich für die Regulation der Cholesterol-Synthese. Hohe Cholesterolkonzentrationen senken die Biosynthese der HMG-CoA-Reduktase, niedrige intrazelluläre Konzentrationen induzieren die Bildung des Enzyms und damit die Cholesterol-Biosynthese.

Natürliche Fermentationsprodukte aus Schimmelpilzen, wie Compactin (A. Endo et al., J. Antibiot. [1976], 29, 1346) und Mevinolin (A.W. Alberts et al., J. Proc. Natl. Acad. Sci. USA [1980], 77, 3957) sind in der Lage, die HMG-CoA-Reduktase kompetitiv zu hemmen.

Inzwischen sind zahlreiche synthetische Derivate dieser Wirkstoffklasse, die einen identischen Lactonring besitzen, als Enzyminhibitoren beschrieben (z. B. G.E. Stokker et al., J. Med. Chem. 28, 347 [1985]).

Die vorliegende Erfindung betrifft neue synthetische Analoga der 3.5-Dihydroxypentansäure in Form des σ -Lactons Ia oder das entsprechende Dihydroxycarbonsäurederivat Ib, Verfahren zu ihrer Herstellung sowie Arzneimittel, die diese Verbindungen enthalten.

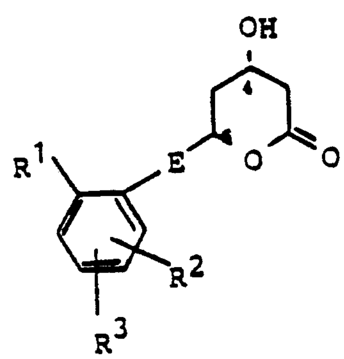

I a

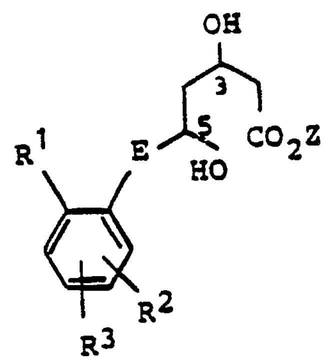

I b

In den Formeln bedeuten:

E einen Alkylen- oder Alkenylen-Rest mit 2 Kohlenstoffatomen,

$R^1$ einen cycloaliphatischen Kohlenwasserstoffrest mit 5 bis 12 Kohlenstoffatomen, der ggf. mit bis zu 4 niederen Alkylgruppen sowie Halogen substituiert ist und ebenfalls ein- oder mehrfach ungesättigt sein kann,

$R^2$,$R^3$, die gleich oder verschieden sein können;

- einen weiteren cycloaliphatischen Kohlenwasserstoffrest mit 3 bis 7 C-Atomen,
- Wasserstoff,
- Halogen,
- Trifluormethyl,
- Alkyl- oder Alkoxy-Rest mit 1 bis 7 C-Atomen, der ggf. noch mit Methylgruppen substituiert sein kann.

Besonders bevorzugter Gegenstand der Erfindung sind Verbindungen, worin die "Brücke" E eine $-CH_2CH_2-$ oder eine -CH=CH-Gruppe, insbesondere die (E)-CH=CH-Gruppe bedeutet.

Unter den Substituenten $R^2$, $R^3$ sind bevorzugt:

Wasserstoff, Halogen, Trifluormethyl, Alkyl- und Alkoxy-Reste mit 1 bis 4 Kohlenstoffatomen.

Bevorzugte Substituenten für $R^1$ sind:

Cycloalkyl- und Cycloalkenyl-Reste mit 5 bis 12 Kohlenstoffatomen.

In den entsprechenden Hydroxycarbonsäuren Ib steht als Rest Z bevorzugt:
Wasserstoff, Alkalimetallion, Ammoniumion oder Alkylgruppen, die sich ableiten von niederen Alkoholen, wie Methanol, Ethanol, Isopropanol oder auch von mehrwertigen Alkoholen (z. B. Glycerin), oder eine Aminogruppe, die sich ableitet von Ammoniak, p-Aminobenzoesäure, ß-Alanin, Ethanolamin, 2-Aminopropanol.

Cycloaliphatische Kohlenwasserstoffreste des Substituenten $R^1$ sind vorzugsweise Cyclopentyl, Cyclohexyl, Cycloheptyl, Cyclododecyl, Cyclopentenyl, Cyclohexenyl, Cycloheptenyl oder Cyclododecenyl.

Cycloaliphatische Kohlenwasserstoffreste der Substituenten $R^2$ und $R^3$ sind bevorzugt Cyclopropyl, Cyclopentyl oder Cyclohexyl.

Niedere Alkylgruppen sind üblicherweise Gruppen mit 1-6 C-Atomen, insbesondere $C_1$-$C_4$, wie Methyl, Ethyl oder Propyl.

Die Alkylreste der Substituenten $R^2$ und $R^3$ sind vorzugsweise Methyl, Ethyl, Propyl oder Butyl. Die Alkoxygruppen sind bevorzugt Methoxy oder Ethoxy.

Unter Halogensubstituenten sind in allen genannten Fällen Fluor, Chlor oder Brom zu verstehen, insbesondere Chlor.

Die obige Definition der erfindungsgemäßigen Verbindungen beinhaltet alle möglichen Stereoisomere sowie ihre Mischungen, bevorzugt das "trans-Lactonring"-Stereoisomer mit der absoluten Konfiguration 4R, 6S; das entspricht der absoluten Konfiguration 3R, 5S im offenkettigen Dihydroxycarbonsäure-Derivat Ib.

Die Herstellung der Verbindungen Ia bzw. Ib ist dadurch gekennzeichnet, daß man entsprechend substituierte Zimtaldehyd-Derivate der Formel II, in welcher $R^1$, $R^2$ und $R^3$ die oben definierte Bedeutung haben und E die -CH = CH-Gruppe bedeutet, mit dem Dianion des Acetessigsäureesters III

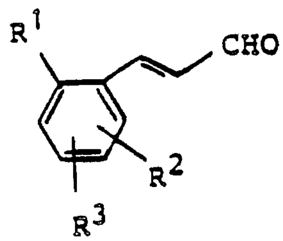

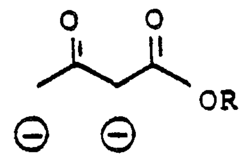

II III

zu der 3-Ketocarbonsäure der Formel IV addiert.

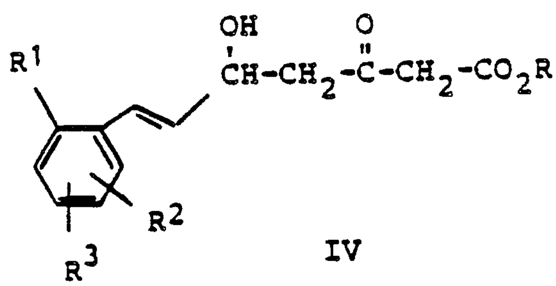

IV

In der allgemeinen Formel IV haben $R^1$, $R^2$ und $R^3$ die in Formel I angegebene Bedeutung; R bedeutet einen niederen Alkylrest.

Die Ketofunktion in der Verbindung der allgemeinen Formel IV wird mit einem milden Reduktionsmittel (z. B. Natriumborhydrid) zum 3.5-Dihydroxycarbonsäureester V reduziert.

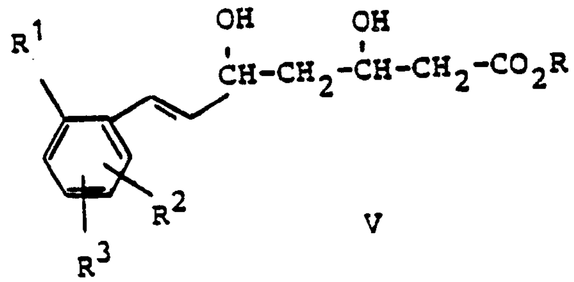

V

Die Substituenten R, $R^1$, $R^2$ und $R^3$ entsprechen der in Formel IV angegebenen Bedeutung.

Die Carbonsäureesterfunktion wird in üblicher Weise alkalisch verseift, daraus nach Ansäuern die freie 3.5-Dihydroxycarbonsäure isoliert.

Diese wird durch Erwärmen in einem inerten Lösungsmittel, wie z. B. Benzol oder Toluol, zum Lacton Ia cyclisiert, worin E für
-CH = CH- steht und nach gängigen Verfahren durch Hydrieren in die
$-CH_2-CH_2-$Brücke umgewandelt werden kann.

**Schema zur Herstellung der Ausgangsmaterialien**

__Weg A:__

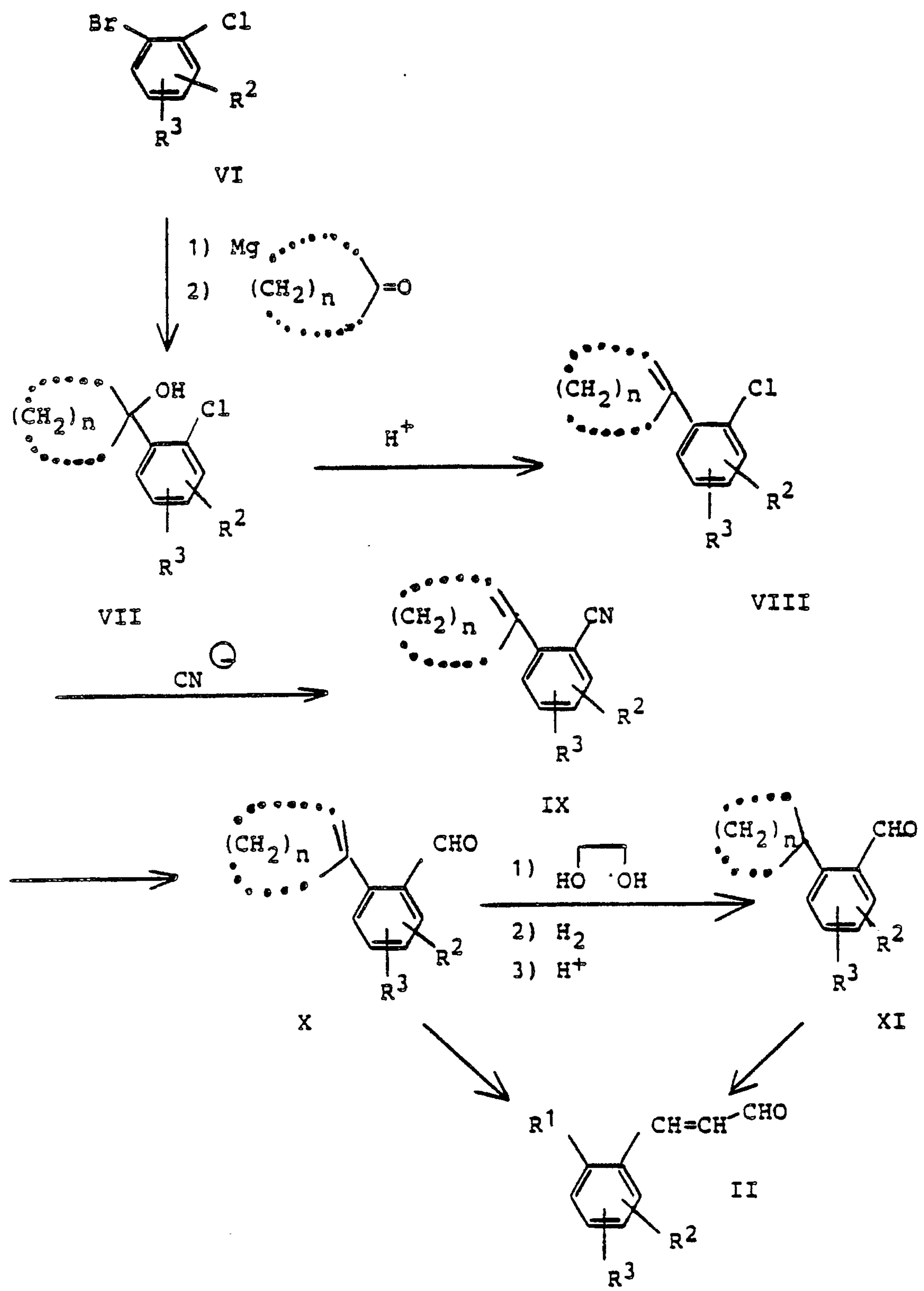

Weg B:

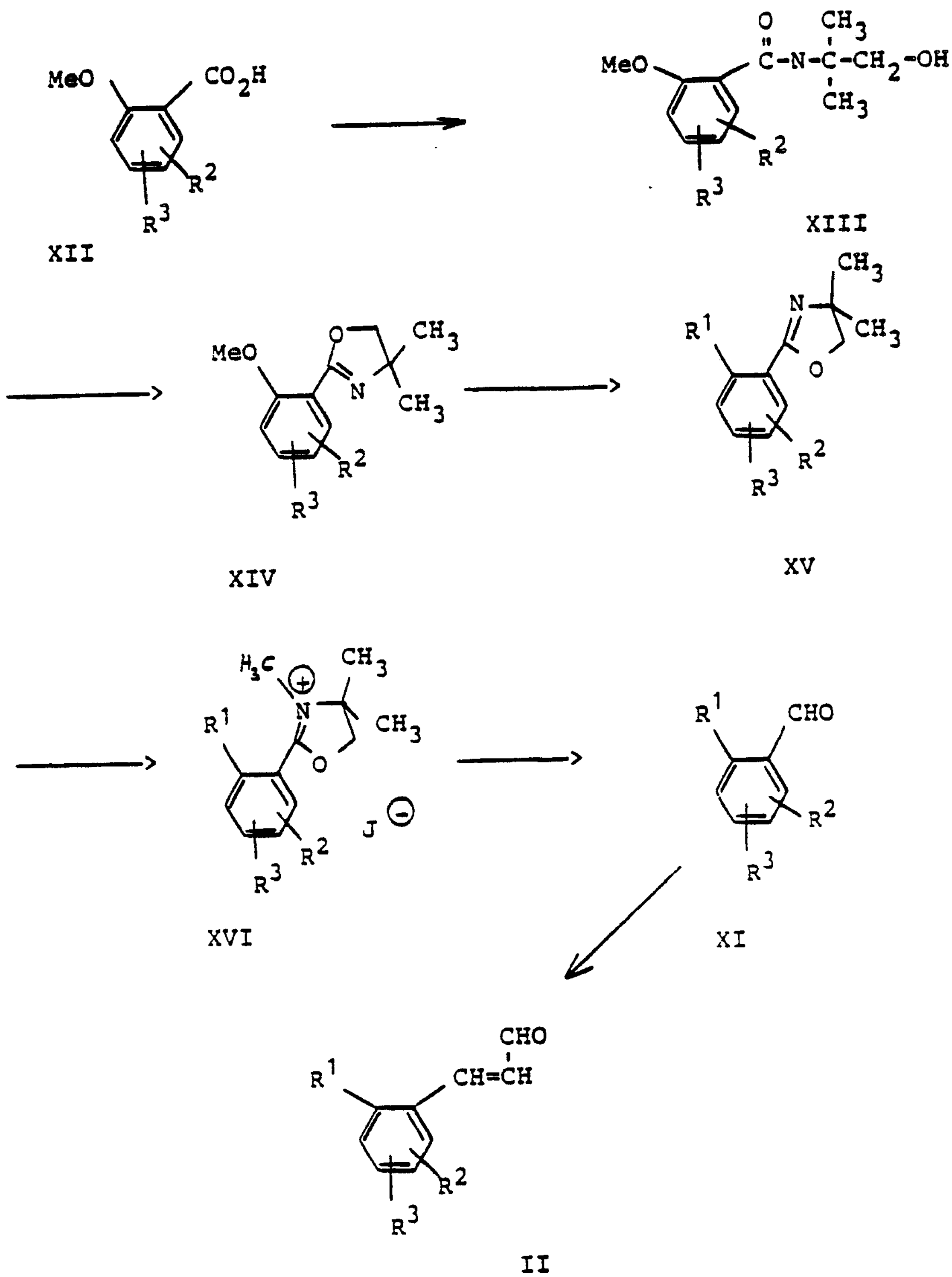

XII

XIII

XIV

XV

XVI

XI

II

Die Verbindungen Ia und Ib der vorliegenden Erfindung erhält man aus dem nach Schema 1 (Weg A) hergestellten Zimtaldehyd-Derivat II, wobei $R^1$, $R^2$ und $R^3$ die zu den allgemeinen Formeln Ia und Ib beanspruchten Substituenten bedeuten.

Das Grignard-Produkt der o-Chlorbrombenzol-Verbindung VI wird mit Cycloalkanonen in wasserfreiem Ethylether oder anderen Lösungsmitteln, wie z. B. höhere Ether (Dibutylether, Tetrahydrofuran) zu dem entsprechenden Cycloalkanol-Derivat VII umgesetzt.

Die Dehydratisierung zum Cycloalken geschieht in wässrigen Säuren, z. B. in Ameisensäure, unter Rückflußbedingungen.

Die Umsetzung zum Benzonitril-Derivat IX erfolgt mit Cyanid-Salzen, wie z. B. Kupfercyanid, in Pyridin bei erhöhter Temperatur (nach: W.E. Parham et al., J. Amer. Chem. Soc. 83, 1751 [1961]).

Das Nitril wird mittels komplexer Metallhydride, wie z. B. DIBAH, in wasserfreiem Ether oder THF als Lösungsmittel zum Benzaldehyd-Derivat X reduziert.

Durch Schützen der Aldehydfunktion als Diacetal oder Cycloacetal, wie z. B. mit Ethylenglycol, Hydrieren unter Edelmetallkatalyse und Abspalten der Schutzgruppe kann in literaturbekannter Weise das Phenylcycloalkyl-System XI aufgebaut werden.

Die Kettenverlängerung zum α, ß-ungesättigten Aldehyd II gelingt z. B. durch Umsetzung der Benzaldehyd-Derivate X und XI mit einem Phosphoniumylid (G. Wittig und R. Haag, Chem. Ber. 88, 1654 [1955]), wobei die bevorzugte Ausführungsform in einem polar aprotischen Lösungsmittel, wie THF oder Dimethylformamid, mit Lithiummethylat oder Butyllithium als Base durchgeführt wird. Dabei werden praktisch reine (E)-Aldehyde erhalten.

Die C-2-Verlängerung kann ebenso mit Diethyl-2-(cyclohexylamino)-vinylphosphonat (W. Nagata et al., Org. Synthesis 53, 44 [1973]) in literaturbekannter Weise durchgeführt werden (W. Nagata, Y. Hayase, Tetrahedron Lett. 4359 [1968] und J. Chem. Soc., C., 460 [1969]).

Die direkte Aldol-Kondensation gelingt ebenso durch Umsetzung des Benzaldehyd-Derivates mit dem Anion eines N-substituierten Ethylidenamins (z. B. Ethylidencyclohexylamin). Die Reaktion, welche bevorzugt in der Kälte in aprotischen Lösungsmitteln, wie THF, durchgeführt wird, liefert ein ß-Hydroxy-ß-phenylpropylidenamin, welches unter gleichzeitiger Dehydratisierung und Imin-Hydrolyse im sauren Medium, wie verd. HCl, den entsprechenden Zimtaldehyd ergibt (G. Wittig, A. Hesse, Org. Synth. Vol. 50, 67).

Ein alternativer Syntheseweg (Weg B) geht auf Arbeiten von Meyers zurück (A.I. Meyers et al., J. Org. Chem. [1978], 43, 1372). Die Schlüsselreaktion dieser Variante ist der Austausch einer Methoxy-bzw. Fluorgruppierung durch eine Grignard-Verbindung.

Dazu werden die 2-Methoxybenzoesäure-Derivate XII mit Thionylchlorid in die entsprechenden Säurechloride und mit 2-Amino-2-Methylpropanol in die Amide XIII umgewandelt. Die Reste $R^2$ und $R^3$ bedeuten die zur allgemeinen Formel I beanspruchten Substituenten.

Das Benzamid XIII kann mit Thionylchlorid/Ether zum Oxazolin-Hydrochlorid überführt werden, aus dem man nach Neutralisation mit wässr. Alkalimetallhydroxiden das Oxazolin XIV isolieren kann.

Die Umsetzung mit der Grignard-Verbindung eines Cycloalkyl-Halogenids in Lösungsmitteln, wie Diethylether oder THF, führt zu den Arylcycloalkyl-Derivaten XV (A.I. Meyers, R. Gabel, E. Mihelich, J.Org.Chem. 43, 1372, [1978]). Versuche mit dem entsprechenden Fluorderivat führten zur selben Zielverbindung.

Quaterinisierung mit Methyljodid in Nitromethan zu XVI und anschließende Reduktion mit milden Reduktionsmittln, wie z. B. Natriumborhydrid, in niederen Alkoholen, bevorzugt Ethanol, und Hydrolyse mit wässriger Säure liefert den gewünschten Aldehyd XI in zufriedenstellenden Ausbeuten.

Die Kettenverlängerung zum α,ß-ungesättigten Aldehyd entspricht den in Weg A beschriebenen Methoden.

Die Reaktion der ungesättigten Aldehyde II mit dem Dianion des Acetessigesters III (z. B. analog S.N. Huckin und L. Weiler, Tetrahedron Lett. [1971], 4835) in einem geeigneten aprotischen Lösungsmittel wie Ether oder THF ergibt den 5-Hydroxy-3-oxoheptensäureester IV.

Die 3-Oxo-Funktion der Ester IV kann mit einem milden Reduktionsmittel in die 3.5-Dihydroxyheptensäure-Derivate V überführt werden, wobei die bevorzugte Ausführung in der Reduktion von IV mit Natriumborhydrid in Methanol als Lösungsmittel besteht. Eine stereo-spezifische, stereo-selektive Reduktion wird mit der Kombination an Trialkylboran und Natriumborhydrid in THF/MeOH bei tiefen Temperaturen (-78 °C) erreicht (vgl. J.E. Lynch et al., Tetrahedron Lett. 28, 1385 - 1388 [1987]).

Die Carbonsäureestergruppe der Verbindung V verseift man in üblicher Weise mittels Alkalimetallhydroxyd und erhält durch Ansäuern der alkalischen Lösung die freie Dihydroxycarbonsäure Ib (Z = H).

Die entstandenen Carbonsäuren können entweder nach üblichen Methoden in pharmazeutisch wirksame Salze überführt oder durch Erwärmen in einem inerten Lösungsmittel, wie Benzol oder Toluol, unter Wasserabspaltung zum Lacton Ia cyclisiert werden.

Die erfindungsgemäßigen Verbindungen der allgemeinen Formel I, bei denen $R^1$, $R^2$ und $R^3$ die zu I angegebene Bedeutung haben und E die Aryl-Lacton-Brücke (E) -CH=CH-bedeutet, können nach allgemein üblichen Methoden, zweckmäßig bei Raumtemperatur mit Wasserstoff in Gegenwart eines Edelmetallkatalysators, wie Palladium oder Platin, in Lösungsmitteln wie Methanol, THF oder Essigester, hydriert werden zu Verbindungen mit einer gesättigten $C_2$-Brücke.

Außer den in den Beispielen beschriebenen Verbindungen lassen sich nach den erfindungsgemäßigen Verfahren die folgenden Verbindungen herstellen:

E-6-[2-(2-Cyclopent-1-enyl)phenyl-ethenyl]-4-hydroxy-3.4.5.6-tetrahydro-2H-pyran-2-on
E-6-[2-(2-Cyclopentyl)-phenyl-ethenyl]-4-hydroxy-3.4.5.6.-tetrahydro-2H-pyran-2-on
E-6-[2-(2-Cyclohex-1-enyl)-phenyl-ethenyl]-4-hydroxy-3.4.5.6-tetrahydro-2H-pyran-2-on
E-6-[2-(2-Cyclohexyl)-phenyl-ethenyl]-4-hydroxy-3.4.5.6-tetrahydro-2H-pyran-2-on
E-6-[2-(2-Cyclohexyl)-6-chlorphenyl-ethyl]-4-hydroxy-3.4.5.6-tetrahydro-2H-pyran-2-on
E-6-[2-(2-Cyclohexyl)-4.6-dichlorphenyl-ethyl]-4-hydroxy-3.4.5.6-tetrahydro-2H-pyran-2-on
E-6-[2-(2-Cyclohept-1-enyl)-phenyl-ethenyl]-4-hydroxy-3.4.5.6-tetrahydro-2H-pyran-on
E-6-[2-(2-Cycloheptyl)-phenyl-ethenyl]-4-hydroxy-3.4.5.6-tetrahydro-2H-pyran-2-on
E-6-[2-(2-Cycloheptyl)-6-chlorphenyl-ethenyl]-4-hydroxy-3.4.5.6-tetrahydro-2H-pyran-2-on
E-6-[2-(2-Cycloheptyl)-4.6-dichlorphenyl-ethenyl]-4-hydroxy-3.4.5.6-tetrahydro-2H-pyran-2-on
E-6-[2-(2-Cyclododec-1-enyl)-phenyl-ethenyl]-4-hydroxy-3.4.5.6-tetrahydro-2H-pyran-2-on
E-6-[2-(2-C3.5-dimethylcyclohexyl)-phenyl-ethenyl]-4-hydroxy-3.4.5.6-tetrahydro-2H-pyran-2-on
E-6-[2-(2-Cyclohexyl)-6-methylphenyl-ethenyl]-4-hydroxy-3.4.5.6-tetrahydro-2H-pyran-2-on
E-6-[2-(2-Cycloheptyl)-4.6-dimethylphenyl-ethenyl]-4-hydroxy-3.4.5.6-tetrahydro-2H-pyran-2-on
E-6-[2-(2-Cyclohexyl)-6-(2-methyl-ethyl)-phenyl-ethenyl]-4-hydroxy-3.4.5.6-tetrahydro-2H-pyran-2-on
E-6-[2-(2-Cyclohexyl)-4.6-dimethylphenyl-ethenyl]-4-hydroxy-3.4.5.6-tetrahydro-2H-pyran-2-on

## BIOLOGISCHE TESTSYSTEME:

### Hemmung der HMG-CoA-Reduktase bei einer Mikrosomenfraktion aus Rattenleber

Zur Bestimmung der HMG-CoA-Reduktase-Aktivität wurden Mikrosomen aus Lebern von Ratten, die 7 Tage bei umgestelltem Tag/Nacht-Rhythmus mit Cholestyramin behandelt waren, mittels Zentrifugation abgetrennt. Die Wirkung der Testsubstanzen bei einer Konzentration von $10^{-4}$ M auf die Bildung von Mevalonat aus (S, R) $^{14}$C-HMG-CoA wurde untersucht. Die Versuchsbedingungen sind im Detail bei Huber, J., Latzien, S., Hamprecht, B.: Hoppe Seylers Z. Physiol. Chem. 354, 1654 [1973] beschrieben.

### $^{14}$C-Acetateinbau in Cholesterin in Rattenhepatozytenmonolayerkulturen

Der Einfluß der Testsubstanzen bei einer Konzentration von $10^{-5}$ M auf den $^{14}$C-Acetateinbau in Cholesterin wurde in Rattenhepatozytenmonolayerkulturen untersucht. Die Abtrennung des neugebildeten Cholesterins erfolgte mittels Säulenextraktion. Bedingungen für Kultur und Extraktion sind bei Pill et al., Fresenius Z. Anal. Chem. 327: 558 - 560 [1987] beschrieben.

Die Verbindungen der Formel Ia bzw. Ib zeichnen sich aus durch starke Hemmung der HMG-CoA-Reduktase. Die 3-Hydroxy-3-methylglutaryl-Coenzym A (HMG-CoA)-Reduktase ist das am stärksten geschwindigkeitsbestimmende Enzym in der Cholesterinbiosynthese (Zafarul, H.B., Brewer, Jr. H.B., Current Topics in Cellular Regulation 20: 139 - 184 [1981] und katalysiert die Synthese von Mevalonat aus HMG-CoA. Die Hemmung dieses Enzyms führt zu einer deutlichen Erniedrigung des Serumcholersterins (Illingworth, D.R., Sexton, G.J., Clin. Invest. 74: 1972 - 1978 [1984]. Die Kombination mit Antihyperlipidämica mit anderen Wirkmechanismen wie z. B. gallensäurebindenden Anionenaustauscherharzen oder Fibraten führt zu einer weiteren Absenkung des Serumcholesterins (Vega, G.L., Grundy, S.M., JAMA 257: 33 - 38 [1987]). Dies ist bei schweren Formen der Hypercholesterinämie erforderlich, um eine ausreichende Serumcholesterinsenkung zu erreichen.

Die erfindungsgemäßen Verbindungen sind Hemmstoffe der HMG-CoA-Reduktase. Sie eignen sich daher alleine und in Kombination mit anderen Antihyperlipidämika zur Prophylaxe und Therapie von Erkrankungen, die durch erhöhtes Serumchloresterin verursacht werden, wie koronare Herzkrankheiten, Atherosclerose, Hypercholesterinämie, Hyperlipoproteinämie und ähnliche Erkrankungen.

Die Erfindung betrifft daher auch pharmazeutische Präparate auf Basis der Verbindungen der Formel Ia bzw. der entsprechenden Dihydroxycarbonsäuren, deren Salze und Ester, sowie die Verwendung dieser

Verbindungen als Arzneimittel, insbesondere zur Behandlung der Hypercholesterinämie.

Die Verbindungen der Formel Ia und Ib werden in verschiedenen Darreichungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 2 mg bis 2.000 mg, vorzugsweise jedoch im Dosisbereich 20 mg bis 500 mg.

Die erfindungsgemäßigen Verbindungen können als Lactone der allgemeinen Formel Ia, in Form der freien Säuren Ib (Z = H) oder in Form pharmazeutisch akzeptabler Salze oder Ester zur Anwendung kommen, und zwar gelöst oder suspendiert in pharmakologisch unbedenklichen organischen Lösungsmitteln wie in ein- und mehrwertigen Alkoholen (z. B. Ethanol, Glycerin, Triacetin), in Ölen (z. B. Sonnenblumenöl), Ethern oder Polyethern oder auch in Gegenwart anderer pharmakologisch unbedenklicher Polymerträger, wie z. B. Polyvinylpyrrolidinon oder anderen pharmazeutisch akzeptablen Zusatzstoffen, wie Stärke, Cyclodextrin oder Polysacchariden.

Die erfindungsgemäßigen Verbindungen können ebenfalls kombiniert werden mit Zusatzstoffen, die Gallensäuren binden, insbesondere mit nichttoxischen, basischen Austauschharzen, die Gallensäuren in einer nichtresorbierbaren Form im Gastrointestinaltrakt binden, wie z. B. Cholestyramin oder Verbindungen aus der europäischen Patentanmeldung EP-A-157 410.

**Herstellung der Vorstufen gemäß Variante A**

**o-(1-Cyclohexenyl)-chlorbenzol**

25.7 g (1.06 mol) Magnesiumspäne wurden mit 191.5 g (1 mol) o-Chlorbrombenzol in 500 ml absol. Ether zur Grignard-Verbindung umgesetzt. Innerhalb 45 Minuten tropfte man 98.4 g (1 mol) Cyclohexanon in 150 ml absol. THF bei 35 °C zu, erwärmte 90 Minuten auf Rückflußtemperatur, kühlte ab und zersetzte mit 130 ml gesättigter Ammoniumchlorid-Lösung. Der Niederschlag wurde abgesaugt, mit THF gewaschen, die organ. Phasen getrocknet und eingeengt. Das Cyclohexanol-Derivat erhielt man in quantitativer Rohausbeute (195 g).

Der Alkohol wurde mit 200 ml 90%iger Ameisensäure 1.5 Stunden unter Rückfluß erhitzt. Nach dem Stehenlassen über Nacht wurde mit Kaliumhydroxid neutralisiert, das abgeschiedene Öl abgetrennt, die wässrige Phase mit Ligroin extrahiert, mit dem Öl vereinigt, getrocknet über Magnesiumsulfat, eingedampft und fraktioniert destilliert.

Ausbeute: 62 %

Siedepunkt: 74 - 75 °C/0.1 mm

$n_D^{25}$ : 1.560 g

**o-(1-Cyclohexenyl)-benzonitril**

Eine Mischung aus o-(1-Cyclohexenyl)-chlorbenzol (19.3 g, 0.1 mol) und 16.4 g (0.18 mol) Kupfer(I)-cyanid in 100 ml N-Methylpyrrolidinon wurde 15 Stunden auf 200 °C erwärmt.

Zur Aufarbeitung wurde das auf 80 bis 100 °C abgekühlte braunschwarze Produkt in eine Lösung aus 40 g Eisen(III)-chlorid, 10 ml konz. Salzsäure und 60 ml Wasser gegeben und die Mischung 0.5 Stunden auf 60 bis 70 °C erwärmt. Die N-Methylpyrrolidinon-Phase wurde abgetrennt, die wässrige mehrmals mit Toluol extrahiert. Die vereinigten organischen Phasen wurden nacheinander gewaschen mit 3N HCl, Wasser, 10%iger Natronlauge, anschließend mit Natriumsulfat getrocknet und eingeengt.

Fraktionierte Destillation lieferte das Benzonitril-Derivat.

Ausbeute: 68 %

Siedepunkt: 90 - 92 °C/0.1 mm

$n_D^{26}$ : 1.5648

**o-(1-Cyclohexenyl)-benzaldehyd**

Zu einer Lösung von 60.5 g (0.33 mol) o-(1-Cyclohexenyl)-benzonitril in 500 ml absol. Ether tropfte man innerhalb 1.5 Stunden unter Kühlung (- 15 °C bis - 10 °C) 360 ml (0.36 mol) 1M DIBAH-Lösung in Methylenchlorid, rührte eine weitere Stunde bei 0 °C, anschließend 3 Stunden bei Raumtemperatur und

zersetzte mit 5N Schwefelsäure. Der Niederschlag wurde abgesaugt, die organische Phase mit NaCl-Lösung gewaschen, getrocknet und eingeengt.

Der Aldehyd wurde ohne weitere Reinigung für die nachfolgenden Stufen verwendet (Rohausbeute: 95 %).

NMR (60 MHz, $CDCl_3$, ppm):
10.25 (1H, s, -CHO); 6.90 - 8.10 (4H, m, aromat. Protonen); 5.70 (1H, m, olef. Proton); 1.05 - 2.50 (8H, m, Cyclohexenyl-Rest).

## o-(1-Cyclohexyl)-benzaldehyd

16.3 g (0.1 mol) o-(1-Cyclohexenyl)-benzaldehyd wurden mit 6.85 g (0.11 mol) Ethylenglycol und 1 g p-TSA in 400 ml Toluol 4 Stunden in einer Dean-Stark-Apparatur erwärmt; danach hatte sich die berechnete Menge Wasser abgeschieden.

Der isolierte geschützte Aldehyd wurde in Ethanol aufgenommen und über $PtO_2$ als Katalysator hydriert. Nach Filtration und Einengen des Lösungsmittels nahm man das Roh-Produkt in 300 ml Dioxan auf, versetzte es mit 80 ml 10%iger wässriger Oxalsäure-Lösung und erhitzte 2 Stunden unter Rückfluß. Nach Extraktion mit Essigester wurde die organische Phase mit Wasser gewaschen, über Magnesiumsulfat getrocknet und eingeengt.

Flash-Chromatographie (Hexan/Essigester 5 : 1) lieferte den Aldehyd in reiner Form in einer Ausbeute von 65 %.

NMR (60 MHz, $CDCl_3$, ppm):
10.28 (1H, s, -CHO); 7.01 - 7.80 (4H, m, arom. Protonen); 1.05 - 3.10 (11H, m, Cyclohexyl).

## o-(Cyclohexyl)-Zimtaldehyd

Zu einer Lösung von 7 ml (5 g, 0.05 mol) Diisopropylamin in 250 ml absol. THF gab man bei 0 °C 35 ml (0.05 mol) Butyllithium in Hexan. Danach wurden 6.25 g (0.05 mol) Ethylidencyclohexylamin bei derselben Temperatur zugesetzt und 15 Minuten nachgerührt.

Nach Abkühlen auf - 78 °C wurden 9.4 g (0.05 mol) o-(Cyclohexyl)-benzaldehyd in 75 ml THF zugetropft und innerhalb 3 Stunden auf Raumtemperatur erwärmt.

Man zersetzte mit Wasser, extrahierte mit Ether, trocknete die org. Phase und engte ein.

Das in quantitativer Rohausbeute erhaltene Imin wurde mit 19 g (0.15 mol) Oxalsäure in 160 ml THF und 35 ml $H_2O$ gelöst und 30 Minuten unter Rückfluß erhitzt. Nach Versetzten mit Wasser wurde mit Ether extrahiert, die organische Phase getrocknet und eingeengt.

Die Reinigung des Zimtaldehyds gelang durch Flash-Chromatographie mit Heptan/Essigester (5 : 1) als Laufmittelgemisch.

NMR (60 MHz, $CDCl_3$, ppm):
9.85 (d, 1H, CHO, J = 8 Hz), 7.05 - 7.70 (m, 4H, arom. Protonen), 7.95 (d, 1H, J = 16 Hz, CH=CH-CHO); 6.75 (dd, 1H, 8 Hz und 16 Hz, CH=CH-CHO); 1.05 - 2.10 und 2.90 (m, 11H, Cyclohexyl-Protonen).

## Beispiel 1

### Synthese der Verbindung

(E)-6-[2-(2-Cycloheptyl)-4,6-dichlorphenyl-ethenyl]-4-hydroxy-3.4.5.6-tetrahydro-2H-pyran-2-on

### Variante B

### 2.4-Dichlor-6-methoxybenzoesäurechlorid

26.0 g (0.117 mol) 2.4-Dichlor-6-methoxybenzoesäure wurden in 50 ml absol. Methylenchlorid gelöst

und nach Zugabe von 0.1 g Dimethylaminopyridin 50 ml Thionylchlorid bei Raumtemperatur zugetropft. Nach 15 Stunden Rühren engte man ein und destillierte den Rückstand.
Ausbeute: 55 %
Sdp. 118 - 124 °C (0.1 Torr)

**N-(2-Hydroxy-1.1-dimethylethyl)-2.4-dichlor-6-methoxybenzamid**

Das oben genannte Benzoesäurechlorid-Derivat (13.9 g, 0.058 mol) wurde in 50 ml absol. Methylenchlorid gelöst und tropfenweise eine Lösung von 11.1 ml (0.116 mol) 2-Amino-2-methyl-1-propanol bei 0 °C zugegeben. Nach einer Stunde Rühren bei Raumtemperatur wurde die Mischung filtriert, das Filtrat mit Wasser, 5 % HCl, 5 % NaOH und konz. Salzlösung gewaschen und eingeengt.
Ausbeute: 90 %

**2-(2.4-Dichlor-6-methoxyphenyl)-4,5-dihydro-4.4-dimethyloxazol**

Zu 2.0 g (6.8 mmol) des Benzamides wurden tropfenweise 10 ml Thionylchlorid unter Eiskühlung gegeben, anschließend versetzte man mit einer Spatelspitze DMAP. Nach 2 Stunden Rühren bei Raumtemperatur gab man absol. Ether zu, rührte kurze Zeit nach und filtrierte das Oxazolin-Hydrochlorid ab. Die gewünschte Verbindung wurde nach Neutralisation mit verdünnter Natronlauge mit Ether extrahiert, die org. Phase getrocknet und eingeengt.
Ausbeute: 87 %
Schmp. 45 °C

**2-(3.5-Dichlor-6-cycloheptyl-phenyl)-4.5-dihydro-4.4-dimethyloxazol**

15.05 g (55 mmol) des oben genannten Oxazol-Derivates wurden in 100 ml absol. THF gelöst und bei Raumtemperatur mit einer 2M Grignard-Lösung (75 mmol) in THF (dargestellt aus Bromcycloheptan und aktiv. Magnesiumspänen) versetzt. Nach 20 Stunden Rühren bei 20 °C tropfte man erneut 60 mmol Grignard-Lösung zu und erwärmte 20 Stunden auf 40 °C. Nach Abkühlen auf 5 °C versetzte man die Mischung mit gesättigter Ammoniumchloridlösung, extrahierte mit Ether, trocknete die org. Phase und engte ein (nahezu quantitative Rohausbeute).
NMR (60 MHz, $CDCl_3$, ppm):
1.30 (6H, s), 3.87 (2H, s), 7.05 - 7.30 (2H, m)

**2-(3.5-Dichlor-6-cycloheptyl-phenyl)-4.5-dihydro-3.4.4-trimethyloxazoliumjodid**

Zu 17 g (50 mmol) des Oxazol-Derivates in 75 ml Nitromethan gab man 4.7 ml (10.65 g, 75 mmol) Methyljodid und erwärmte 10 Stunden auf 100 °C. Nach Einengen im Vakuum wurde der verbleibende Rückstand mit wenig Ether gewaschen und abgesaugt. Ohne weitere Reinigung wurde das Oxazolium-Jodid-Derivat für die nächste Reaktion eingesetzt.
Ausbeute: 78 %
Schmp: 145 °C (Zersetzung)

**2.4-Dichlor-6-cycloheptyl-benzaldehyd**

Zu einer Suspension des Oxazolium-Jodid-Derivats (17.2 g, 45 mmol) in 175 ml absol. Ethanol gab man portionsweise 2.5 g (65 mmol) Natriumborhydrid und rührte 3 Stunden bei Raumtemperatur. Nach Versetzen mit 300 ml 2N Salzsäure erwärmte man eine Stunde auf 80 °C, verdünnte nach dem Abkühlen mit Wasser und extrahierte mehrmals mit Ether. Die organ. Phasen wurden mit konz. Salzlösung und Wasser gewaschen, getrocknet und eingeengt. Der Aldehyd konnte nach Flash-Chromatogrphie (Hexan/Essigester 8 : 1) in reiner Form erhalten werden.
Ausbeute: 56 %
NMR (60 MHz, $CDCl_3$, ppm):

10.52 (1H, s, CHO), 7.05 - 7.40 (2H, m)

**2.4-Dichlor-6-cycloheptyl-zimtaldehyd**

5.2 g (20 mmol) Diethyl-2-(cyclohexylamino)-vinylphosphonat (W. Nagata et al., Org. Synthesis 53, 44 [1973]) wurden zu einer Suspension von 0.48 g (20 mmol) Natriumhydrid in 30 ml absol. THF bei 0 °C gegeben. Bei derselben Temperatur ließ man 30 Minuten später eine Lösung von 4.6 g (17 mmol) des 2.4-Dichlor-6-cycloheptyl-benzaldehyds in 30 ml absol. THF zufließen. Dabei stieg die Temperatur von 0 °C auf 30 °C an. Man rührte 3 Stunden nach, goß auf Eiswasser und extrahierte mit Ether.

Der Etherrückstand wurde in 100 ml THF gelöst, 6 g Oxalsäure in 100 ml Wasser zugesetzt und 1.5 Stunden auf Rückflußtemperatur erwärmt. Tetrahydrofuran wurde im Vakuum abdestilliert, die wässrige Phase mit Essigester extrahiert. Die org. Phase wurde nacheinander mit 2N Salzsäure, Wasser, Bicarbonatlösung und Wasser gewaschen, getrocknet und eingeengt.

Die Reinigung erfolgte durch Flash-Chromatographie (Hexan/Essigestesr 5 : 1).

Schmp. 90 - 92 °C

NMR (60 MHz, $CDCl_3$, ppm):

10.85 (1H, d, CHO, J = 8 Hz), 7.65 (1H, d, CH=CH-CHO, J = 15 Hz), 6.48 (1H, dd, CH=CH-CHO, J = 15 Hz, J = 8 Hz).

**(E)-7-(2.4-Dichlor-6-cycloheptyl-phenyl)-5-hydroxy-3-oxo-6-heptensäuremethylester**

0.9 ml (8 mmol) Acetessigsäuremethylester wurden bei O °C zu einer Suspension von 0.31 g (13 mmol) Natriumhydrid in 30 ml absol. THF zugetropft. Nach 15 Minuten Rühren gab man innerhalb 30 Minuten 7.5 ml (13 mmol) einer 1.7 molaren Lösung von Butyllithium in Hexan zu.

Zur der entstandenen gelben Lösung wurden 2.4 g (8 mmol) 2.4-Dichlor-6-cycloheptyl-zimtaldehyd in 20 ml absol. THF gegeben, eine Stunde bei 0 °C nachgerührt und anschließend mit 50 ml halbkonzentrierter Salzsäure zersetzt. Die Reaktionsmischung wurde mit Wasser verdünnt, mit Ether extrahiert, die Ether-Extrakte neutral gewaschen, getrocknet und eingeengt.

Das Rohprodukt wurde durch Flash-Chromatographie (Heptan/Essigester 3 : 1) gereinigt.

Ausbeute: 68 %

Schmp: 78 - 79 °C

NMR (300 MHz, $d_6$-DMSO, ppm):

1.40 - 1.80 (m, 12H, Cycloheptyl-Rest), 2.75 (2H, d, J = 6 Hz), 3.00 (1H, m), 3.23 (s, 1H), 3.62 (s, 3H), 4.6 (m, 1H), 5.78 (1H, dd, J = 15 und 6 Hz), 6.48 (1H, d, J = 15 Hz), 7.24 und 7.36 (2H, 2d, J = 2 Hz).

**(E)-7-(2.4-Dichlor-6-cycloheptyl-phenyl)-3.5-dihydroxy-6-heptensäuremethylester**

Zu 0.8 g (2 mmol) des oben beschriebenen 3-Ketoesters in 15 ml absol. Methanol gab man portionsweise bei 0 °C 100 mg Natriumborhydrid, rührte die Lösung 2 Stunden unter weiterer Kühlung, zersetzte mit 6N Salzsäure, extrahierte mit Ether, trocknete die org. Phase und engte ein.

Ausbeute: 87 %

**(E)-6-[2-(2-Cycloheptyl)-4.6-dichlorphenyl-ethenyl]-4-hydroxy-3.4.5.6-tetrahydro-2H-pyran-2-on**

Zu einer Lösung von 0.65 g (1.5 mmol) des oben beschriebenen Dihydroxyesters in 20 ml Ethanol gab man 3 ml (3 mmol) einer 1M Natronlauge und rührte 2 Stunden bei Raumtemperatur. Nach dem Ansäuern mit verd. Salzsäure extrahierte man mit Ether, trocknete und engte ein. Der Rückstand wurden in 30 ml Toluol gelöst und 12 Stunden bei 60 °C gerührt, um die Dihydroxycarbonsäure vollständig zum Lacton zu cyclisieren.

Das Gemisch von trans- und cis-Lacton (Verhältnis 2 : 1) wurde durch Flash-Chromatographie (Laufmittel Methylenchlorid/Aceton 20 : 1) vollständig aufgetrennt.

(±) trans-Isomer:

NMR (300 MHz, DMSO, ppm):
7.40 und 7.29 (2H, 2d, J = 2 Hz), 6.62 (1H, d, J = 15 H), 5.85 (1H, dd, J = 15 und 8 Hz), 5.32 (1H, m), 5.24 (1H, d, J = 3 Hz, OH), 4.20 (1H, m), 2.95 (1H, m), 2.72 (1H, dd, J = 16 und 6 Hz), 2.40 - 2.55 (1H, m), 1.4 - 2.02 (14H, m).

Die Charakterisierung der cis- und trans-Isomeren erfolgt über NMR-Spektroskopie.

(±) cis-Isomer:

NMR (300 MHz, DMSO, ppm):
7.39 und 7.28 (2H, 2d, J = 2 Hz), 6.60 (1H, d, J = 15 HZ), 5.82 (1H, dd, J = 15 und 8 Hz), 5.15 (1H, d, J = 3 Hz, OH), 5.02 (1H, m), 4.20 (1H, m), 2.95 (1H, m), 2.85 (1H, dd, J = 16 Hz und 6 Hz), 2.32 (1H, dd, J = 16 Hz und 4 Hz), 1.4 - 1.85 (14H, m).

Die Auftrennung der ± trans- bzw. cis-Isomeren erfolgt in üblicher Weise mit optisch aktiven Basen.

**Beispiel 2**

In analoger Weise wie in Beispiel 1 beschrieben werden die folgenden ± trans-Verbindungen erhalten:

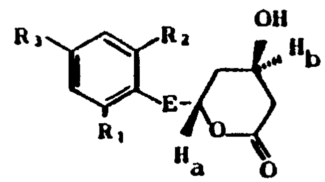

(±)

**Tabelle 1**

| | R₁ | R₂ | R₃ | E | Schmp. °C | δ (ppm) Olefin.Prot. (E-Brücke) | $H_a$ | $H_b$ | OH |
|---|---|---|---|---|---|---|---|---|---|
| a) | 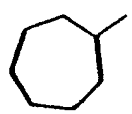 | Cl | Cl | -CH=CH- | Oel | 6.61 (1,J=15 Hz) 5.85 (dd,J=15 u.6 Hz) | 5.25-5.35 | 4.15-4.23 | 5.25 (d,J=3 Hz) |
| b) | 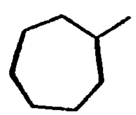 | Cl | H | -CH=CH- | Oel | 6.65 (d,J=16 Hz) 5.81 (dd,J=16 u.6 Hz) | 5.28-5.37 | 4.15-4.25 | 5.24 (d,J=3 Hz) |
| c) | 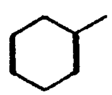 | Cl | Cl | -CH=CH- | 103-104 | 6.60 (d,J=16 Hz) 5.88 (dd,J=16 u.8 Hz) | 5.25-5.35 (m) | 4.16-4.23 | - |

(±) 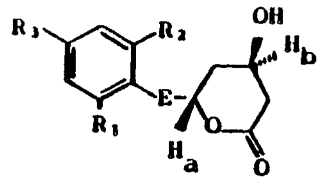

Tabelle 1

| | $R_1$ | $R_2$ | $R_3$ | E | Schmp. °C | δ (ppm) Olefin.Prot. (E-Brücke) | $H_a$ | $H_b$ | OH |
|---|---|---|---|---|---|---|---|---|---|
| d) | 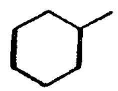 | Cl | H | $-CH=CH-$ | 90-92 | 6.65 (d,J=16 Hz) 5.82 (dd,J=16 u.8 Hz) | 5.24-5.34 | 4.18-4.24 | - |
| e) | 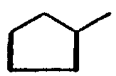 | H | H | $-CH=CH-$ | Oel | 7.04 (d,J=16 Hz) 6.15 (dd,J=16 u.7 Hz) | 5.25-5.34 (m) | 4.15-4.23 (m) | 5.22 (breit) |
| f) | 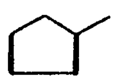 | H | H | $-CH_2CH_2-$ | Oel | - | 4.55-4.65 (m) | 4.10-4.16 | 5.10 (breit) |

(±) 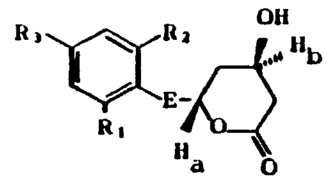

Tabelle 1

| | R₁ | $R_2$ | $R_3$ | E | Schmp. °C | δ [ppm] Olefin.Prot. (E-Brücke) | $H_a$ | $H_b$ | OH |
|---|---|---|---|---|---|---|---|---|---|
| g) | 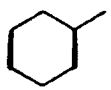 | H | H | -CH=CH- | Oel | 7.00 (d,J=16 Hz) 6.15 (dd,J=16 u.7 Hz) | 5.25-5.35 (m) | 4.14-4.21 (m) | 5.21 J=3 Hz) |
| h) | 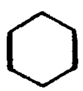 | H | H | $-CH_2CH_2-$ | Oel | - | 4.54-4.64 (m) | 4.10-4.16 (m) | 5.11 J=3 Hz) |
| i) | 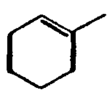 | H | H | -CH=CH- | Oel | 6.81 (d,J=16 Hz) 6.10 (dd,J=16 u.8 Hz) | 5.25-5.35 (m) | 4.32-4.41 (m) | - |

(±)

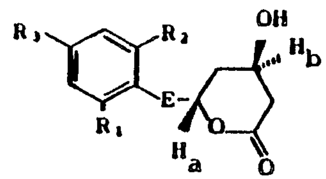

Tabelle 1

| R₁ | $R_2$ | $R_3$ | E | Schmp. °C | δ (ppm) Olefin.Prot. (E-Brücke) | $H_a$ | $H_b$ | OH |
|---|---|---|---|---|---|---|---|---|
| j) 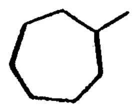 | H | H | -CH=CH- | Oel | 6.98 (d,J=16 Hz) 6.13 (dd,J=16 u.8Hz) | 5.25-5.30 (m) | 4.14-4.22 (m) | 5.20 (d, J=3 Hz) |
| k) 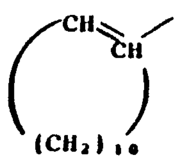 | H | H | -CH=CH- | Oel | 6.82 (d,J=16 Hz) 6.20 (dd,J=16 u. 8Hz) | 5.15-5.25 (m) | 4.10-4.21 (m) | 5.12 (d, J=3 Hz) |
| l) 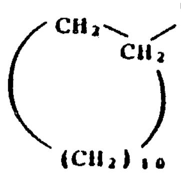 | H | H | $-H_2C-CH_2-$ | Oel | - | 4.58-4.67 | 4.10-4.17 | 5.14 (d,J=2 Hz) |

(±) 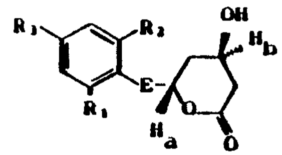

**Tabelle 1**

| | R₁ | R₂ | R₃ | E | Schmp. °C | δ [ppm] Olefin.Prot. (E-Brücke) | Ha | Hb | OH |
|---|---|---|---|---|---|---|---|---|---|
| m) | 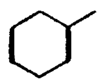 | $CH_3$ | H | -CH=CH- | Öl | 6.68 (d,J=16 Hz) 5.66 (dd,J= 16 u. 6.5 Hz) | 5.27-5.35 | 4.20 | 5.24 (d,J=3 Hz) |
| n) | 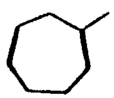 | $CH_3$ | $CH_3$ | -CH=CH- | 114-115°C | 6.65 (d,J=16 Hz) 5.62 (dd,J= 16 u. 6.5 Hz) | 5.22-5.32 | 4.19 | 5.25 (breit) |
| o) | 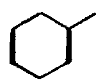 | $H_3C$ CH- $H_3C$ | H | -CH=CH- | 102-104°C | 6.82 (d,J=16 Hz) 5.63 (dd,J=16 u. 6 Hz) | 5.38-5.48 | 4.45 | - |
| p) | 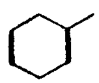 | $CH_3$ | $CH_3$ | -CH=CH- | 129-131°C | 6.65 (d,J=16 Hz) 5.63 (dd,J=16 u. 6.5 Hz) | 5.23-5.32 | 4.18 | 5.25 (breit) |

**Ansprüche**

1. Verbindungen der Formel Ia und Ib

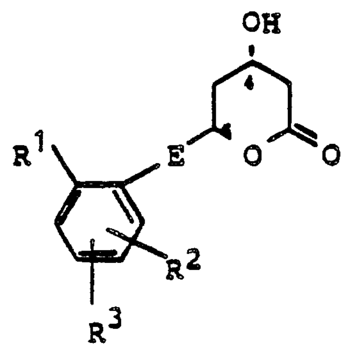

I a

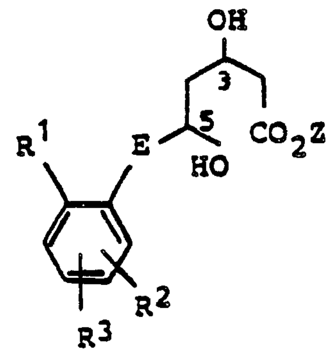

I b

in denen
E eine Alkylen- oder Alkenylen-Rest mit 2 Kohlenstoffatomen,
$R^1$ einen cycloaliphatischen Kohlenwasserstoffrest mit 5 bis 12 Kohlenstoffatomen, der ggf. mit bis zu 4 niederen Alkylgruppen sowie Halogen substituiert ist und ebenfalls ein- oder mehrfach ungesättigt sein kann,
$R^2$, $R^3$ die gleich oder verschieden sein können:
- einen weiteren cycloaliphatischen Kohlenwasserstoffrest mit 3 bis 7 C-Atomen,
- Wasserstoff,
- Halogen
- Trifluormethyl,
- Alkyl- oder Alkoxy-Rest mit 1 bis 7 C-Atomen, der ggf. noch mit Methylgruppen substituiert sein kann. Z Wasserstoff, ein Alkalimetallion oder ein Ammoniumion bedeuten,

Ester und Amide der freien Carbonsäure, sowie deren stereoisomere Formen.

2. Verbindungen gemäß Anspruch 1, in denen $R_1$ Cyclopentyl, Cyclohexyl, Cycloheptyl, Cyclododecyl, Cyclopentenyl, Cyclohexenyl, Cycloheptenyl oder Cyclododecenyl bedeuten.

3. Verbindungen gemäß Anspruch 1, in denen $R_2$ Wasserstoff, Halogen, Methyl oder Isopropyl bedeuten.

4. Verbindungen gemäß Anspruch 1, in denen $R_3$ Wasserstoff, Halogen oder Methyl bedeuten.

5. Verfahren zur Herstellung der Verbindungen der Formel Ia und Ib

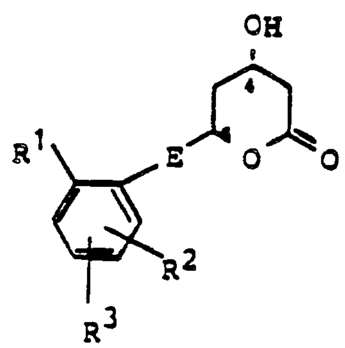

I a

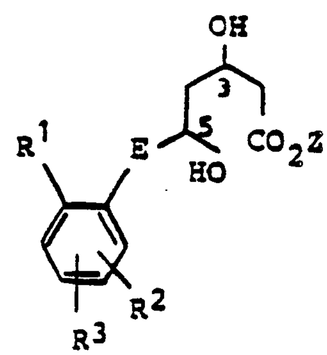

I b

in denen
E eine Alkylen- oder Alkenylen-Rest mit 2 Kohlenstoffatomen,

$R^1$ einen cycloaliphatischen Kohlenwasserstoffrest mit 5 bis 12 Kohlenstoffatomen, der ggf. mit bis zu 4 niederen Alkylgruppen sowie Halogen substituiert ist und ebenfalls ein- oder mehrfach ungesättigt sein kann,
$R^2$, $R^3$ die gleich oder verschieden sein können:
- einen weiteren cycloaliphatischen Kohlenwasserstoffrest mit 3 bis 7 C-Atomen,
- Wasserstoff,
- Halogen
- Trifluormethyl,
- Alkyl- oder Alkoxy-Rest mit 1 bis 7 C-Atomen, der ggf. noch mit Methylgruppen substituiert sein kann.

Z Wasserstoff, ein Alkalimetallion oder ein Ammoniumion bedeuten,
Ester und Amide der freien Carbonsäure, sowie deren stereoisomere Formen, dadurch gekennzeichnet, daß man in an sich bekannter Weise eine Verbindung der Formel II,

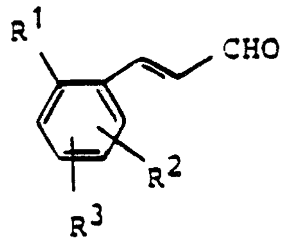

II

in der $R_1$, $R_2$ und $R_3$ die angegebene Bedeutung haben, mit einem Dianion des Acetessigesters III,

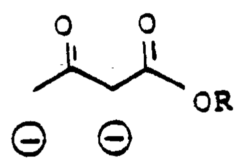

III

in der R eine niedere Alkylgruppe bedeutet, zu einer Verbindung der Formel IV umsetzt,

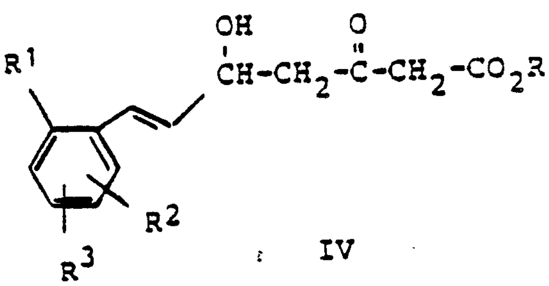

IV

in der R, $R_1$, $R_2$ und $R_3$ die angebende Bedeutung haben,
und anschließend die Ketogruppe reduziert sowie gegebenenfalls die Estergruppierung verseift, die 3,5-Dihydroxycarbonsäure derivatisiert bzw. zum Lacton der Formel Ia cyclisiert und gewünschtenfalls die Gruppe E - CH = CH - hydriert und die stereoisomeren Verbindungen auftrennt und isoliert.

6. Arzneimittel, enthaltend eine Verbindung gemäß Anspruch 1 bis 4 sowie unbedenkliche Träger- und Hilfsstoffe.

7. Verwendung von Verbindungen gemäß Anspruch 1 bis 4 zur Behandlung von Stoffwechselerkrankungen.

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

Nummer der Anmeldung

EP 89109371.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 232 997 (MERCK & CO.INC.) * Ansprüche 1,7,9,10; Seite 6, Zeilen 10-14 * -- | 1,5,6 | C 07 D 309/30<br>C 07 C 69/732<br>C 07 C 59/48<br>A 61 K 31/365 |
| A | DE - A1 - 3 530 797 (HOECHST AG) * Ansprüche 1,4,6 * -- | 1,5,6 | |
| A | DE - A1 - 3 525 256 (SANDOZ-PATENT-GMBH) * Ansprüche 1,9; Seite 12, Absätze 3,4 * -- | 1,5,6 | |
| A | CHEMICAL ABSTRACTS, Band 107, Nr. 5, 3. August 1987, Columbus, Ohio, USA HOECHST AG "Preparation of 3-demethylmevalonic acid derivatives as prophylactic and therapeutic agents for arteriosclerosis and hypercholesteremia." Seite 680, Spalte 2, Zusammen- | 1,5,6 | |

RECHERCHIERTE SACHGEBIETE (Int. Cl.4)

C 07 D 309/00
C 07 C 69/00
C 07 C 59/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6
Unvollständig recherchierte Patentansprüche: -
Nicht recherchierte Patentansprüche: 7
Grund für die Beschränkung der Recherche:

*(Verfahren zur therapeutischen Behandlung des menschlichen oder tierischen Körpers, Art. 52(4), EPÜ)*

| Recherchenort | Abschlußdatum der Recherche | Prüfer |
|---|---|---|
| WIEN | 12-07-1989 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

# EUROPÄISCHER TEILRECHERCHENBERICHT

Nummer der Anmeldung

-2-

EP 89109371.8

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.4) |
|---|---|---|---|
| | fassung Nr. 39 611c<br>& Jpn. Kokai Tokkyo Koho JP 62 51 641 (87 51 641) 06 March 1987<br>---- | | |
| | | | RECHERCHIERTE SACHGEBIETE (Int. Cl.4) |